# EUROPEAN PATENT APPLICATION

(11) **EP 2 388 023 A1**
(43) Date of publication of application: **23.11.2011**
(21) Application number: 11006266.8
(22) Date of filing: 18.05.2007
(51) Int. Cl.: A61L 27/54, A61L 27/12, A61K 38/08

(54) **Composites on a basis of bioceramics and biodegradable or non biodegradable polymers, containing antibiotics for tissue restitution and products therefrom**

(30) Priority: 18.05.2006 BR PI0602372
(62) Divisional of application: 07719328.2
(71) Applicant: Universidade Federal de Minas Gerais, Belo Horizonte MG (BR)
(72) Inventor: Dario Sinistrerra Millan, Ruben, Minas Gerais CEP 31310-390 (BR); Cortes, Segura, Maria, Esperanza, Minas Gerais CEP 31310-390 (BR); Souza, Dos Santos, Robson, Augusto, Minas Gerais, CEP 31310-340 (BR); Pataro, André, Luiz, Minas Gerais, CEP 30310-010 (BR); De Oliveira, Michele, Fabiane, Minas Gerais, CEP 30770-190 (BR); Paz Lopes, Miriam, Teresa, Minas Gerais CEP 31340-360 (BR); De Marco, Turchetti Maia, Regina Maria, Minas Gerais, CEP 31310-370 (BR)
(74) Representative: Lorente Berges, Ana

(57) **Abstract**

Composites based on bioceramics containing calcium salts like Ca₅(PO₄)₃OH, CaCO₃, CaSO₄, CaSO₂, Ca₃(PO₄), CaOSiO₂(NO₃)₂, Ca₁₀(PO₄)₆(OH)₂, biodegradable or non biodegradable polymers, and antibiotics, and uses of such composites in the process of tissue restitution.

## Description

### FIELD OF THE INVENTION

This invention is characterized by the preparation of composites on a basis of bioceramics containing calcium salts like Ca₅(PO₄)₃OH, CaCO₃, CaSO₄, CaSO₂, Ca₃(PO₄), CaOSiO₂(NO₃)₂, Ca₁₀(PO₄)₆(OH)₂, natural and/or synthetic biodegradable polymers containing antibiotics in the form of suspensions, cosmetics, grafts, and uses of such compositions in the process of tissue restitution. This technology is related to a methodology used in the production of composites containing antibiotics that can be used as grafts in humans and as veterinary medicinal products. We claim moreover the plasticity of the composites when blended, for example, not limiting, in the proportion of 75:25 (Osteosynt® bioceramics / biodegradable or non biodegradable polymers), mimicking the inorganic:organic proportion of the bones. The biodegradable polymers may be either PLA, PGA or PLGA, and their corresponding blends, and the non biodegradable, like PMMA. This feature allows easy handling and molding in various forms, depending on the requirements of the tissue being replaced, and calcium phosphates can also be used.

Another feature of the present invention is the use of tetracyclines in the composites, in concentrations varying from 0,001% to 5%, effective as an anti-inflammatory drug therefore showing better biocompatibility when compared to other materials found in state-of-the-art techniques.

The bioceramics/polymer composites and/or cements claimed in this document display the induction properties of the repairing dentine, formation of compact dentine bridge as well as a matrix with favorable geometry for mineral deposition with superior features as compared to those presented by other materials found in state-of-the-art techniques. This material induces a more effective tissue repair and promotes a histological response compatible with the natural tissue regeneration process.

### BACKGROUND OF THE INVENTION

State-of-the-art techniques offer various types of materials for grafts, like plates, pins, and threads made of metal or ceramic that can be used for osteosynthesis. However, these materials present a number of problems due to their high level of elasticity as compared to that of natural bones therefore reducing the peripheral strength of the bones exposed to excessive stress after restoration, Lemaitre, et al., patent US6906516, 2005.

In particular, metallic grafts release metallic ions that may cause harm the host and, in some cases, cause cancer, delay in the process of bone restitution therefore requiring another surgical procedure to remove the graft.

Lemaitre, et al., patent US6905516, 2005 proposed a technology which uses a bone substitute with controlled anatomical shape and macroporous structure made from chemically consolidated cement phosphocalcic materials. Said materials can be molded and consolidated ex-vivo and then grafted. However, the use of bioceramics and biodegradable polymer composites is not claimed in such invention.

Other procedures offered for bone reconstruction by state-of-the-art techniques include the use of biodegradable and bioabsorbable polymers, like lactic acid polymers (PLA), glycolic acid (PGA) and their respective co-polymers (PLGA). Those polymers are hydrolyzed and have, in most cases, a good level of biocompatibility. Another advantage of the biodegradable polymers is that they do not have to be removed after been grafted, a procedure for metallic grafts, as seen in patent US 6,875,442, Holy et al., 2005.

Another procedure described in the literature involves the use of calcium hydroxide as remineralization inducing agent in exposed dentine or pulp capping. However, the calcium hydroxide releases a high concentration of calcium ions in the first 24 hours resulting in intense superficial necrosis while the bioceramics:polymer composites herein claimed produce calcium ions from a matrix with favorable geometry for mineral deposition for up to 30 days, leading to a more effective tissue repair, and producing a histological response compatible to the natural process of tissue restitution.

The present invention is also related to the inhibition, reduction, regulation or prevention of connective tissue degradation. This state-of-the-art technique uses non-antimicrobial tetracycline, most specifically associated to its capacity to inhibit the production and activity of proteinases in biological systems where such inhibition is required, in quantities that include the synergetic blend of tetracycline and biphosphonates. The proteinases involved in the disease process or inflammatory response include the matrix metalloproteinasis (MMP).

Patents US466897 (1987), Golub et al., and US4704383, McNamara, et al., (1987) describe methods for the inhibition of mammal collagenase with the use of tetracyclines.

Patent US2002192279, Sebastian et al., describes the production method of composites containing antibiotics where a salt can be plastically deformed. Such material consist of at least one cationic component of protonated antibiotics like tetracycline, one inorganic anionic component like sulfates, or organic like sulfonates, and/or fat acid esters as connecting components for the composites. However, the composites of this invention comprise the use of bioceramics and biodegradable polymers mainly, while the tetracycline is presented as micro or nanoencapsulated.

In his patent US 5,290271 (1994), Jernberg, G. R. claims surgical grafts and a methodology for the controlled and sustainable release of polymer encapsulated chemotherapeutic agents and/or calcium phosphate structures from a porous matrix, aiming at increasing the cell absorption of those drugs. Said technology claims the use of grafts made from biodegradable polymers but differently from the claim herein contained.

Patent US5321017, Golub, et al., 1994, describes a composition containing fluorobiprofen and tetracycline for the reduction of tissue destruction and bone loss. The patent claims the use for the treatment of rheumatoid arthritis and other medical conditions related to tissue destruction resulting from the excessive activity of the metalloproteinases (or metalloproteases), combining concentrations of antimicrobial effective against the metalloproteinases, but not as antimicrobial agents. In addition, patent US459135 (1995), by the same inventor, describes a bone loss reduction drug which combines indometacyn and tetracycline

Patent US6541037 by Lee, et. al., 2003 describe a release device made from low crystalline hydroxiapatite and a biologically active agent to be used in bones, central nervous system, intramuscular, subcutaneous, intraperitoneal and ocular sites.

Collagenase enzymes are typically present in mammals. This enzyme is produced by tissues and cells and usually degraded by a collagen during the remodeling of connective tissues. It is the most important structural protein of the connective tissues like bones, tendons, skin and the gingiva. However, the excessive production of collagenase leads to the pathological destruction of those tissues. Said production has been observed in a number of diseases such as hyperparathyroidism, diabetes, periodontal disease and rheumatoid arthritis. Those processes may result in excessive bone reabsorption, ulceration of the cornea, destruction of conjunctive tissues associated with arthritis and rupture of gingiva collagen fibers, as well as bone loss associated to the presence of periodental pockets.

Tetracyclines, like doxicycline and minocycline, are effective broad-spectrum bacteriostactic agents. These agents act directly against anaerobic micro-organisms (Fusobacterium sp), aerobic gram-positive and gram-negative, besides rickettsias, mycoplasms, chlamydias, micobacterias, spirochaetas and some protozoans. Tetracyclines also display anti-inflammatory properties including the inhibition of: immunoglobulin secretion by lymphocytes B, leucocytes chemotaxy (neutrophyles), prostaglandin synthesis, metalloproteinases activity (eg. collagenases and gelatinases), bone reabsorption (anti-proteolitic effect or effect over osteoclasts), phospholipase A2 and expression of nitric oxide synthesis. It also favors conjunctive re-insertion. (Seymor e Heasman, Pharmacological control of periodental disease. II. Antimicrobial agents. J Dentistry, 23, 1:. 5-14, 1996).

Increasing tissue collagenase activity both in diabetic humans and rats was identified in gingiva and skin extracts (Ramamurthy and Golub, Diabetes increases collagenase activity in extracts of rat gingiva and skin, J Periodontal Res, 18, 23-30, 1983) and gingival tissue culture (Ramamurthy, et al., J. Periodontal Res., 9, 199-206 (1974); Golub, et al., J. Dent, Res., 57, 520-525, 1978; Kaplan, et al., J. Dent. Res., 61, Special Issue, 275, 1982). Acceleration of collagen rupture mediated by excessive collagenase resulting from this systemic disease was also identified in tests with animals. (Bissada, et al. Periodontics, 4, 223 (1866).

Reports on the efficacy of tetracycline for the treatment of rheumatoid arthritis are also found in the literature. (Brown, et al., Comp. Pathol. Zoo Animals, eds. Montali & Migaki, Smithsonian Institution Press, 259-266 (1980)).

State-of-the-art patents describe the role of tetracyclines as collagen growth modulator, and tetracyclines associated individually to bioceramics or to biodegradable polymers. However, neither of those patents claims their use in bioceramics and biodegradable or non biodegradable polymer composites, which is the basic characteristic of the invention herein described.

The literature also describe the use of calcium hydroxide as exposed dentine and pulp capping remineralization inducer. However, calcium hydroxide liberates high concentrations of calcium ions in short periods of time, which results in intense superficial necrosis. On the other hand, the bioceramics:polymer composites claimed in this document produce calcium ions from a matrix with a more favorable geometry for osteoid deposition during longer periods of time, between two and four weeks, thus leading to a more effective tissue repair and promoting a histological response that is more compatible with the normal process of tissue restitution.

Therefore, the invention herein claimed has the necessary strategy for the achievement of easy-handling composites, as well as mechanical resistance, with induction capacity for the neoformation of high density osteoid tissue. These results are obtained by the composites claimed in this invention, using the Osteosynt bioceramics as described in patent BR PI 9104220-8, and by the biodegradable polymers, like PLGA, PGA and PLA and their blends, as a non-limiting example.

### DETAILED DESCRIPTION OF THE INVENTION

Composites made from bioceramics and the various biodegradable polymers- PLA and PLGA - in various molar proportions with cold and hot moldable characteristics, although preferably in the molar proportion of 75,0:25,0% bioceramics:polymer. The moldable proportions of the composite allow its use as bone fillings and in buccofacial surgeries. The composites herein claimed are characterized by their high level of biocompatibility.

Another feature of the invention is its capability to generate a compact dentine bridge free from tunnels on the surface when placed in the pulp capping of the teeth, thus proving its applicability for tissue reconstruction. Those features indicate the advantage of the composites in comparison with calcium hydroxide, another material commonly found in state-of-the-art techniques for the same purpose.

Another characteristic of the invention is the development of structural parts when the material is associated to non degradable polymers, like the PMMA, or as an injection substance associated to a biocompatible carrier like the calcium alginate, or the hydroximethyl cellulose, for example.

Achievement of calcium salt bioceramics materials, generically known as hydroxiapatite basically occurs through a physical process of commercial calcium salt syntherization, as that of the tricalcium phosphate and/or by means of humidity via (JARCHO) or by purification at approximately 1200°C with variations clearly defined for porosity and density in the various stages under an atmosphere of N₂/H₂O after the mixing and compaction of the components at 500 P, with control of time of exposure, addition and reduction and variations of solubilization.

Addition and blending of calcium phosphates obtained through humidity via (JARCHO) in the proportions suggested, physical process of syntherization at approximately 1200°C, after compaction at 500 P, with pressure variation of N₂/H₂O with variations allowing the production of a less dense, more soluble ceramics.

The production of those substances is also achieved with the 24-hour micronic purification of bone diaphasis crushed (or not) with ethylenediamine - 1:20-hour distilled water x6 bath to obtain the mineral matrix; 1:12-hour chlorophormium/methanol bath - 24-hour 0,6 M (1:70) chlorydric acid bath - maintenance of the pH 5.5 with a 0.2 M phosphate buffer solution, bath - distilled water x6 bath - bath in 2 mol of CaCl₂ 24 h 4°C, bath in 0,5 mol EDTA 4°C during 24 hours - bath 8 in mol LICI 24 h 4°C - bath H₂O 24 h 55°C-lyophilization OGC to obtain organic matrix and morphogenetic activities of the proteic growth factors of the cell matrix.

### EXAMPLES

***Preparation of the hydrated PLGA*/*bioceramics composites.***

Composites made from Osteosynt bioceramics with granulometry of 60x80 mesh (HA1) and 60x40 Mesh (HA2) dispersed in a biodegradable polymer matrix of hydrated PLGA 50:50. To optimize their mechanical properties, the composites were prepared according to the following proportions: 95,5:0,5: 99,0:1,0; 98,5:1.5; 95,0:5,0; 90.0:10,0; 85,0:15,0; 80,0:20,0; 75,0:25,0 % w/w bioceramics/PLGA.

Both the bioceramics and the PLGA were properly weighed, respected the above-mentioned percentages, and then quantitatively transferred to a glass plate followed by homogenization. The composites were stored in hermetically sealed containers and kept in a dryer. Samples of this composite were subjected to chemical-physical characterization.

The description of the procedures, which uses 25% PLGA and 75% bioceramics with the two granulometries, shows the achievement of a moldable and flexible composite which is adequately prepared to match buccofacial and orthopedic surgical needs in general, as well as the requirements of pulp recapping. It is worth noting that the composite is hardened after a few hours. However, by adding drops of dichloromethane and crushing it in an agate mortar, or heating it at 75°, it is possible to regain the previously mentioned consistency.

The composites and the original materials were characterized through the absorption spectography of the infrared region. The main absorption bands and tentative attributions for bioceramics HA1 and HA2 are shown in Table 1. From the spectrum above, we can observe that, as the absorption regions in IV are equivalent, HA1 and HA2 have the same functional groups. Therefore they differ only in relation to their granulometries, the former being more pulverized that the latter. Table 2 shows the main bands of the PLGA polymer.

**Table 1- Main absorption bands in IV for HA 1 and HA 2**

| **Absorption bands (cm⁻¹)** | **Attribution (Tentative)** |
|---|---|
| 3500 | O-H Stretch |
| 1050-990 | P-O Stretch |
| 650-500 | P-O stretch |

**Table 2- Main absorption bands in IV for the hydrated PLGA**

| **Absorption (cm⁻¹)** | **Attribution (tentative)** |
|---|---|
| 3500 | O-H Stretch |
| 1800-1720 | C=O Stretch |
| 1480-1430 | C-H Deformation |
| 1257-1155 | Aliphatic eater |

The analysis of the spectrum in the infrared region of the HA1 and hydrated PLGA composites, and the composites 99,5:0,5, 99,0:1,0, 98,5:1,5, 95,0:5,0 % w/w of HA 1/PLGA shows that as the proportion of polymer is increased in the bioceramics, the band in 1100cm -1 is expanded, which suggests an Increased interaction between the polyester, groups PO4-3, and the bioceramics. The main bands of the spectrum in the infrared region of the HA1, HA2 and hydrated PLGA composites are shown in Table 3.

**Table 3- Main absorption bands in IV for composites of various percentages of HA 1/PLGA and HA 2/PLGA.**

| **Absorption bands (cm⁻¹)** | **Attribution (tentative)** |
|---|---|
| 3600 | O-H Stretch |
| 1640-1535 | C=O Stretch |
| 650-500 | P-O Stretch |
| 1480-1430 | C-H Deformation |
| 1050-990 | P-O Stretch |

Thermogravimetric (TGA) and differential exploratory calorimetric (DSC) analysis were used to characterize the original materials and the composites. The analysis of the TG curves shows thermostability of the HA1 between 25-750°C. The TG curve of composites of different percentages of bioceramics/hydrated PLGA where bioceramics is used is HA1. It is observed that the composites whose percentage of PLGA is 0.5%, 1.0% and 1.5% show thermal stability between 25-760°C. However, as the percentage of PLGA gradually increases, the composite shows higher mass loss compared to what occurs with HA1. Such losses may result from the decomposition of the PLGA, once HA1 is thermostable under this temperature. Therefore, as the percentage of PLGA increases, more mass loss is observed in the composite. The same is observed for the TG curves of the HA2 and PLGA composites.

The DSC curve of the HA1 shows an exothermal peak around 108°C, corresponding to a change of the HA1 stage. The analysis of the DSC curves of composites 99,5:0,5, 99,0:1,0, 95,0:5,0 % w/w de HA 1/PLGA shows that they have an endothermal peak around 220°C corresponding to the change of stage of the composites and the absence of the peak at 108°C of the HA 1. The analyis of the DSC curves of composites 90,0:10,0, 85,0:15,0, 80,0:20,0 % w/w HA 1/PLGA shows peaks around 151°C, 221°C e 304°C, the first two being endothermal and the last exothermal in all composites. The first event can be associated to the process of thermal decomposition. In the DSC curves of composites 99,5:0,5, 95,0:5,0, 90,0:10,0, 85,0:15,0, 80,0:20,0, 75,0:25,0 % w/w HA 2/PLGA, an endothermal event around 160°C can be observed, suggesting a more intensive change in stage, coherent with the increased percentage of PLGA in the composite. Those results indicate a difference in the thermal behavior of the HA2 composites, as compared to that of the HA1 composites.

The patterns of HA1 and HA2 diffraction are similar. The samples display a polycrystalline behavior. The analysis of the PLGA profile shows that it also has a semicrystalline behavior, with an amorphous halo of 20 to 30° 2θ. The behavior of the composites was similar to that of the bioceramics and PLGA composites. The analysis of the X-Ray diffractograms of HA1/PLGA composites in various proportions shows that the increased percentage of PLGA results in a better organization of the crystalline net for the composite, which becomes more similar to the HA1 profiles. This is observed in the composite 85,0:15,0 % w/w HA 1/PLGA. These results suggest a possible increase of the hydrophobic character of the composite compared to that of the free PLGA. The analysis of those diffractograms shows that the increased percentage of PLGA results in a better organization of the crystalline net of the composite, which becomes more similar to the profiles of the HA2. This is observed in sample 85,0:15,0 % w/w of HA 2/PLGA as well as in the HA1 composites.

The micrography of composites HA1 and HA2 suggest that HA1 has a smaller granulometry than HA2 while both samples display irregular morphology and a high level of porosity. On the other hand, the PLGA is amorphous, has a plastic appearance and is devoid of pores. The increased percentage of PLGA in the formulation of the composite reduces its porosity, as it is covered by the PLGA and is devoid of pores. This is easily observed in the 98,5:1,5 % w/w HA 1/PLGA and HA 2/PLGA composites. It is also evidenced that in almost all HA1 and HA2 related composites the PLGA is amorphous with a cylindrical morphology. Said change is due to the fact that the bioceramics has pores and indentations, therefore being able to give the PLGA the desired form. Those results suggest that the ceramics matrix serves as a scaffold for the reshaping of the polymer, coherent with the organization process identified in the X-ray diffractograms when the proportion of the polymers in the HA1 and HA2 matrices is increased.

***Preparation of the bioceramics*/*PLGA and bioceramics*/*PLA composites***

Composites made from Osteosynt bioceramics with granulometry of 60x80 mesh (HA1) dispersed in a biodegradable polymer matrix of PLGA 50:50 and PLA. The composites had the following proportions: 85,0:15,0, 80,0:20,0 and 75,0:25,0 % w/w bioceramics/polymer. The bioceramics and the sprayed PLGA were weighed and quantitatively transferred to a mortar. 0.200ml of dichloromethane was added for each gram of composite. The blend was homogenized until a partial evaporation of the solvent was obtained. The same procedure was followed to obtain the PLA composite. Samples of that composite were submitted to chemical-physical characterization and further in vitro and in vivo biological tests.

The results of the PLGA and PLA polymers and the composites with HA2 are presented in this part of the document. It is important to stress that those are new polymer samples that required full chemical-physical characterization. The procedure described in example 1 was used to obtain a moldable composite with 25% polymer (PLGA or PLA) and 75% bioceramics. It was observed that after 12 hours of exposition to air the composites displayed a brittle structure. Besides, they are not rigid as expected immediately after preparation. To obtain this rigidity each gram of composite was submitted to a 3-minute silicon immersion heating at 75 °, While warm, the composite can be easily molded.

Table 4 shows the main absorption bands of PLA and its tentative attribution. The spectrum suggests a more crystalline sample as compared to the PLGA spectrum. Table 5 shows the main absorption bands and their tentative HA2 and PLA composite attribution.

**Table 4 - Main absorption bands in the infrared for the PLA.**

| **Absorption band (cm⁻¹)** | **Attribution (Tentative)** |
|---|---|
| 3500 | O-H Stretch |
| 3090 | C-H Stretch |
| 1760 | C=O Stretch |
| 1480-1430 | C-H Deformation |
| 1250-1000 | Aliphatic ester |

**Table 5 - Main absorption bands in the IV for composites of various percentages of HA 2/PLA.**

| **Absorption band (cm⁻¹)** | **Attribution (Tentative)** |
|---|---|
| 3500 | O-H Stretch |
| 1640-1635 | C=O Stretch |
| 1480-1430 | C-H Deformation |
| 1050-990 | P-O Stretch |

The analysis of the TG curves of the composites indicate their lower thermal stability as compared to that of the free PLGA, once their thermodecompositions occur between 250 and 350°C. The gradual increase of the PLGA in the composition of the composite results in increasing mass loss of 15, 20 and 25% for 85,0;15,0, 80,0:20,0 and 75,0:25,0 % w/w HA 1/PLGA composites. The analysis of the TG curve of the PLA shows that its thermodecomposition occurs between 275-310°C. The behavior of those composites is similar to that of the PLGA composites, although their thermodecompositions normally occur between 250-275°C.

The DSC curve for the PLA presents endothermal events around 50°C and 297°C, the former associated to vitreous transition and the latter to polymer decomposition. The DSC curves for all the composites display an endothermal peak around 262°C corresponding to PI A thermodecomposition. Laterally the event associated to the vitreous transition of the polymer is not observed.

The analysis of the crystallographic profile of the PLA shows it is also amorphous, presenting an amorphous halo of 15 to 25° 2θ. The behavior of the composites was similar to that of the bioceramics and PLGA composites.

The analysis of the micrographies indicates that both polymers are amorphous, presenting a plastic appearance and few pores. The increased percentage of PLGA in the formulation of the composite reduces its porosity, as it is covered by PLGA, which Is devoid of pores. The micrographies with PLA composites amplified 200x, show behavior similar to PLGA composites, that is, an increase in the percentage of PLA while the pores are reduced. Interestingly, after heating, the 75:25% composite displays morphology quite similar to that of the pure HA1, which suggests a process for the preparation of the moldings with composites, without changing the basic morphology of the HA2.

***Preparation of the bioceramics*/*PLGA and bioceramics*/*PLA composites with tetracycline* (*TCN*)**

The composites are made with Osteosynt bioceramics with granulometry of 60x80 mesh (HA2), dispersed in PLGA 50:50 and PLA biodegradable polymer matrixes. The proportion used is 75.0:25.0 % w/w bioceramics/polymer, added of 0.01, 0.1 and 10 % w/w TCN. The bioceramics, the sprayed PLGA and the tetracycline were weighed and quantitatively transferred to a mortar. 0.200ml of dichloromethane was added for each gram of composite. The blend was homogenized until a partial evaporation of the solvent was obtained. The same procedure was followed to obtain the PLA composite. Spectrum analysis of all composites in region IV showed that the gradual Increase of the percentage of tetracycline does not result in changes In the spectrum of bioceramics.

The absorption bands of the tetracycline and its tentative attribution are shown in 1 able 9 below. The main composite absorption bands are shown in Table 10..

**Table 9 - Main absorption bands in IV for tetracycline**

| **Absorption band (cm⁻¹)** | **Attribution (Tentative)** |
|---|---|
| 3400-3300 | N-H Stretch |
| 3200-3000 | O-H Stretch |
| 1740 | C=O Stretch |
| 1600-1650 | C=C Stretch |
| 1200 | C-O Stretch |
| 995-985 | C-H Deformation |

**Table 10 - Main absorption bands in IV for composites containing TCN.**

| **Absorption band (cm⁻¹)** | **Attribution (Tentative)** |
|---|---|
| 3400-3300 | O-H Stretch |
| 1750 | C=O Stretch |
| 1100-1000 | P-O Stretch |
| 995-985 | C-H Deformation |

The TG curve of the TCN indicted thermal stability between 25-200°C and later significant mass loss, around 220 °C, associated to the total thermodecomposition of the tetracycline. The composites had a mass loss of around 25%, and the gradual increase of tetracycline results in the higher thermal stability of the composites compared to the thermal stability of the composite without tetracycline.

The DSC curve of the TCN shows a 225 °C endothermal and a 235° C exothermal events. Those results indicate the occurrence of a merger followed by thermodecomposition. The analysis of the DSC curve of the composites indicates absence of thermal events and formation of more stable composites, like the formation of chelates between the tetracycline and calcium ion present in the bioceramics. The difratogram of tetracycline indicates that this drug has a crystalline crystallographic pattern, between 2 and 40 29 presenting characteristic peaks.

The analysis of composites containing tetracycline displays a semicrystalline behavior, similar to that of the composite without that drug. The difratograms for the PLA composites containing 1.0, 0.1, 0.01% tetracycline suggest that they have a behavior similar to those containing PLGA> The analysis of the micrographies obtained for pellets prepared with the composites containing PLGA tetracycline 1.0% and 0.1% amplified 20 and 100x. It was observed that the latter display irregular morphology and a great level of porosity. The micrographies obtained for pellets prepared with the PLA composites containing tetracycline 1.0% and 0.1%, amplified 20 and 100x also displayed irregular morphology and great level of porosity.

***Texture analysis***

Gaseous adsorption is an important technique for the characterization of the so-called texture properties of the materials. The determination of the specific superficial area and of the distribution of pore sizes of samples was made with the automatic system of adsorption of the Autosorb - Quantachrome NOVA 1200 equipment, which supplies both absorption and deabsorption data. The samples were decarbonated during one hour at 80 0C before each assay. The error percentage supplied by the equipment is 5%. The specific superficial area measures were based on the Brunauer-Emmett-Teller (BET) - BET Equation principle and the analysis of pore sizes and total volume was obtained through the Barrett, Joyner & Halenda (BJH) method.

This analysis is valid only for measures of the mesopores and micropores of the materials. Macropores are beyond the limits of ascertainment of this characterization technique. The samples analyzed were: (A) Composite containing 75% bioceramics with granulometry of 100x80 mesh and 25% hydrated PLGA; (B) composite containing 75% bioceramics with granulometry of 80x60 mesh and 25% hydrated PLGA, (C) BRS commercial composite; (D) composite containing 75% bioceramics with granulometry of 80x60 mesh and 25% PLGA pressed under 2 ton/ m² during 5 minutes.

Table 11 displays the results of texture analysis of the materials: (A) Composite containing 75% bioceramics with granulometry of 100x80 mesh and 25% hydrated PLGA; (B) composite containing 75% bioceramics with granulometry of 80x60 mesh and 25% hydrated PLGA; (C) BRS commercial composite; (D) composite containing 75% bioceramics with granulometry of 80x60 mesh and 25% PLGA pressed under 2 ton/ m² during 5 minutes.

Those results show that the samples did not display substantial difference of texture with specific superficial area and very low total volume of pores. The composite displayed specific superficial area and total volume of pores higher than that of the commercial composite. The commercial material had average pores diameters (micropores) smaller than those of the synthetic materials (mesopores).

**Table 11 - Values of the specific superficial area, total volume and average diameter of pores**

| **Samples** | **A_{BET}** | **V_{T}** | **dp** |
|---|---|---|---|
| | | | **(A)** |
| | **(m²/g)** | **(10⁻³cm³/g)** | |
| A | 1,0 | 1,2 | 36 |
| B | 1,0 | 1,4 | 36 |
| C | 9.6 | 10,7 | 23 |
| D | 13,7 | 12,8 | 36 |

***Preparation of pellets for biological tests***

5 mm diameter pellets were prepared with those samples under pressure of 250 kg/m² during 20 seconds. The composites were submitted to chemical-physical characterization and further in vitro and in vivo biological tests.

Percent variations of 20:80 to 80:20 between the bioceramics and the biodegradable polymers (PLGA or PLA) were produced to optimize the mechanical properties of composites. Due to the various proportions tested for bioceramics and polymers, the proportion of 3:1 was established (3 parts of bioceramics and one part of polymer), ensuring similarity with the inorganic and organic proportion of bone constitution. Groups with and without tetracycline (TCN), first with concentration of 4% and later 0.01 % were formed.

The composites were prepared and conformed in pellets, first under pressure of 200 kg/m². Later it was observed that pellet formation under that pressure resulted in the reduction of pellet macropores therefore hindering water absorption. Various tests were performed under pressure of 250 kg/m² for sample standardization. The comparison of hand with hydraulic pellet formation at 250 kg/m² did not display macroscopic changes.

**Cytotoxicity tests of composites**

Tests to determine the in vitro biological cytotoxicity of the biomaterials evaluate the effects of the contact of those materials with conjunctive tissue cells of rats (L929 cell fibroblasts). The direct contact test performed was the ASTM F-813, ISO-10993-5

***Cultivation of animal cell lineage***

Cell lineages were cultivated in 5% bovine fetal serum medium in culture bottles in humid oven kept at a temperature of 37°C, containing 2.5% CO₂. Before reaching confluence, the cells were tripsinized to be subcultivated for immediate use.

***Direct contact method- ASTM F-813***

Monolayers of the conjunctive tissue L929 cells suspension of rats were placed on 35mm diameter Petri plates and incubated for approximately 48 hours. After achieving the proper confluence, the liquid culture medium was aspirated and replaced with a fresh culture medium and bovine fetal serum. Samples of negative control (high density poliethylene of the positive control (latex) and of the materials previously sterilized with ethylene oxide (Curar - Centro de Esterilização Especializada Ltda., Belo Horizonte - MG) were placed in direct contact with the monolayer of the cell. The cell cultures were incubated for 24 hours with the samples to evaluate their cytotoxic properties. The toxio components present in the materials can affect the cells located at various distances of the samples. After delimiting the contact areas of the samples on the outside surface of the plates, the pellets were removed and the monolayer was colored with a violet crystal solution. The zone index (ZI) corresponding to the area or clear zone where the cells were not colored (Box 1) and the lysis index (LI), which indicates the percentage of degenerated or affected cells within the toxicity zone (Box 2) were set up for each plate after the microscopic analysis. The zone index (ZI) and the lysis index (LI) are related to provide the response index (RI) through the mathematical formula RI=ZI/LI.

Table 12 displays the lysis indexes, affected zone and response indexes of fibroblasts compared to composites, according to the indexes presented in Boxes 1 and 2 obtained through the direct contact test (ASTM F-813, ISO-10993-5). The composites were grouped as follows: N1 (PLGA:HA 50:50); N2 (PLA:HA 50:50); N3 (PLGA:HA 20:80 + TCN 4%) and N4 (PLA:HA 20:80 + TCN 4%).

The analysis of the data obtained for this assay evidences the higher cytotoxicity of samples N3 and N4, which contain 4% TCN and a higher proportion of HA as compared to polymers.

**Box 1 - Description of Provoked Lysis and Corresponding indexes (LI)**

| IL | ***Description*** | Classification |
|---|---|---|
| 0 | No lysis | None |
| 1 | Less than 20% of affected zone | Light |
| 2 | Between 20 and 39% of affected zone | Mild |
| 3 | Between 40 e 59% of affected zone | Moderate |
| 4 | Between 60 e 80% of affected zone | Severe |
| 5 | More than 80% of affected zone | Severe |

**Box 2 - Description of the Zone Affected and Corresponding Zone Indexes (IZ)**

| ZI | Description | Classification |
|---|---|---|
| 0 | No zone under or around the sample | None |
| 1 | Limited zone under sample | Light |
| 2 | Zone with less than 0.5cm around sample | Mild |
| 3 | Limited zone, between 0.5 and 1.0 cm around sample | Moderate |
| 4 | Zone with more than 1.0cm around sample, but not covering the whole plate. | Severe |
| 5 | Zone covering the whole plate | Severe |

Table 12 - *In vitro* evaluation of the cytotoxicity of composites in a culture of fibroblasts (L929 cell line) obtained through a direct contact test (ASTM F-813, ISO-10993-5). Characteristics of materials: solid, weight between 45 (N₃ e N₄) and 50 mg (N₁ e N₂), slightly dense.

**Table 12 - In vitro evaluation.**

| Plate # | Sample identification | Zone index (ZI) | Lysis index (LI) | Response Index (RI) RI= ZI / LI | Additional observations |
|---|---|---|---|---|---|
| 1 | HD poliethylene | 0 | 0 | 0/0 | Control - |
| 2 | HD poliethylene | 0 | 0 | 0/0 | |
| 3 | HD poliethylene | 0 | 0 | 0/0 | |
| 4 | Latex | 2 | 5 | 2/5 | Control + |
| 5 | Latex | 2 | 5 | 2/5 | |
| 6 | Latex | 2 | 5 | 2/5 | |
| 7 | N₁ | 1 | 3 | 1/3 | |
| 8 | N₁ | 1 | 3 | 1/3 | |
| 9 | N₁ | 1 | 2 | 1/2 | |
| 10 | N₂ | 1 | 1 | 1/1 | |
| 11 | N₂ | 1 | 1 | 1/1 | |
| 12 | N₂ | 1 | 1 | 1/1 | |
| 13 | N₃ | 3 | 3 | 3/3 | |
| 14 | N₃ | 3 | 3 | 3/3 | |
| 15 | N₃ | 3 | 3 | 3/3 | |
| 16 | N₄ | 2 | 2 | 2/2 | |
| 17 | N₄ | 2 | 2 | 2/2 | |
| 18 | N₄ | 2 | 2 | 2/2 | |

| | | | | | |
|---|---|---|---|---|---|
| Level of cytotoxicity in ascending order latex (positive control) >N₃>N₄>N₁>N₂. | | | | | |

***Biocompatibility tests performed Preparation of composites***

The composites were made with bioceramics (75%) dispersed in a biodegradable polymer matrix (25%), similar to the proportion of the inorganic and organic bone constitution, associated or not to tetracycline.

The materials tested were conformed in 7mm diameter pellets, approximately, under pressure of 250 kg/m² and sterilized with ethylene oxide (Curar - Centro de Esterilização Especializada Ltda., Belo Horizonte - MG). The following groups were formed: N1-PLGA:HA; N2-PLGA:HA + 0,1% tetracycline; N3-PLA:HA ; N4-PLA:HA + 0,1% tetracycline; C1-PLGA; C2-PLA ; C3-HA.

To do the experiments, 45 Swiss isogenic rats with approximately 8 weeks of age were used. The rats were supplied by the Bioterism Center of the Institute of Biological Sciences (CEBIO) of the Federal University of Minas Gerais (UFMG), and kept *ad libitum* at the Animal Room of the Immunology and Immunophysiology Laboratory of the Institute of Biological Sciences (ICB) - UFMG, being fed with standard feed and strained water. The experiments followed the strict recommendations of the UFMG ethics committee.

To perform the implantation (Seq. 1), the animals were properly anesthetized with a 10ml physiological saline solution - 1ml solution and 10% ketamine (Agener, São Paulo, SP) and 1ml Calmium^{®} (Agener, São Paulo, SP), a muscle relaxant, painkiller and sedative. The dosage, between 0.2 and 0.4ml, was applied via intraperitoneal injection.

The anesthetized animals were subjected to trichotomy of the dorsal region, after the local application of antiseptic ethanol (70%). A dorsal medial incision between the iliac spines with divulsion of subcutaneous tissue with cranial and caudal direction was performed. The composites were introduced in the interscapular region and in a caudal direction. Two or three pellets were implanted in each animal.

Catgut 5-0, a ½ CR 2,0cm curved needle (Brasuture, S.S. Grama, SP) and double stitches were used for the incision suture, followed by local application of an antiseptic solution. All procedures were performed in a laminar flow chamber.

After 1, 7, 14, 21, 28, and 56 days, the rats were sacrificed by overdose of anesthesia to avoid unnecessary suffering, maintain the integrity of adjacent tissues and grafts and the absence of blood, therefore allowing the identification and restitution of tissues.

A median incision was performed on the dorsal region of rats to allow for the removal of the composites and subsequent histological analysis.

Immediately after the removal of the pellets, the tissues under the grafts were removed from the rats and placed in a formalin buffer at 10% during 24 hours. The tissues were then incubated for one-hour in ascending dehydrating solution of ethanol (80%, 95% e 100%) followed by incubation in xylol to remove the excess of alcohol and to obtain a translucent material. After this treatment, the material was twice immersed in paraffin wax, cut in 5µm microtomes and colored with hematoxylin-eosin for further analysis under optical microscope.

The pellets of the materials implanted in rats were removed within above mentioned times and conditioned for six hours in a 2% glutaraldehyde solution in sodium phosphate buffer pH 7.4, followed by alcohol dehydration overnight at 37°C.

The micrographies were obtained at 20.0 KV, using a JEOL-JSM-840A equipment of the Institute of Exact Sciences (ICEX) of the Federal University of Minas Gerais (UFMG).

Table 13 displays the results of the biocompatibility tests of the composites implanted In rats (n=45) with groups N1: PLGA:HA; PLGA:HA + 0.1% tetracycline; N3: PLA:HA ; N4: PLA:HA + 0,1% tetracycline; C1: PLGA; C2: PLA ; C3: HA.

The composites and the control groups produced acute inflammation between days one and seven day, when chronic inflammation was installed, mainly in the composite grafts. On day twenty-eight the inflammation receded almost completely, leaving only light residues in few groups.

The rats implanted with the PLGA:HA composite enjoyed a longer inflammatory period. The group receiving pure hydroxiapalite presented the lowest evidence of inflammation and in a shorter period of time.

The inflammatory reaction of the host is a natural response to the damage and the presence of an alien object. The magnitude and duration of the inflammatory process has a direct impact on the biostability and biocompatibility of the biomaterial (Kao & Lee, 2001).

Various factors contribute a biological response of a material in the implanted environment: chemical formulation, size, form, implantation site and lifespan of the specific graft implanted in the tissue.

Blood cells, neutrophils, macrophages and giant cells play an important role in the Incorporation of the graft. Lymphocytes, plasma cells, natural killers and macrophages are actively involved in the antigenic complex or immunological reactions associated to the incorporation of natural transplants or grafts. Immunological responses are usually of minor importance when the material used is synthetic polymers, metal, ceramics and carbon, or their composites.

Neutrophils and macrophages are related to wound repair and the healing processes, as occurs in the elimination of bacteria that may cause infection in the graft area thus threatening the implantation process. Neutrophils are mainly involved in the acute inflammatory stage and are relatively short-lived compared to the macrophages (and their derivatives) involved in chronic inflammation and in the healing stage (Ziats et al., 1988).

The composite micrographies evidenced the presence of a layer of conjunctive tissue covering the whole area of the graft. This condition was found in most of the pellets between days one and fifty six. Also, conjunctive tissue partially covering the graft and presence of some cells probably adsorbed on the surface of the pellet.

After performing the microscopic analysis of some pellets from day one, this layer of covered tissue was partially or fully removed. The composite pellets containing PLGA displayed more adhesion and, therefore, more resistance towards removal of that tissue. This condition was due to the higher degradation speed of the PLGA polymer, where the byproducts possible had a higher amount of hydrogen connections in the interface with the surrounding tissue.

The pellet of the PLGA:HA composite, removed 13 days after implantation displayed: a) composite degradation and presence of conjunctive tissue and b) indication of the presence of adhered cells, or, probably, adsorbed on the surface of the composite.

During the process of bone neoformation from biomaterial, a scaffold is required as it acts as a temporary matrix for the dependent-anchorage of osteoblasts besides its intrinsic filling function. It can also act as a carrier for soluble and insoluble factors modulating local cell function.

**Table 13: Biocompatibility of the composites in rats.**

| Groups | ***Day 1*** | **I.I.** | ***Day* 7** | **I.I**. | ***Day 13*** | **I.I**. | ***Day 21*** | **I.I**. | ***Day 28*** | **I.I**. | ***Day 56*** | **I.I**. |
|---|---|---|---|---|---|---|---|---|---|---|---|---|
| N1 | +++ | PMN | +++ | PMN and LP | +++ | LP and PMN | - | - | - | - | + | LP |
| N2 | ++ | PMN | +++ | PMN and LP | + | LP and PMN | + | LP | L | - | + | LP |
| N3 | +++ | PMN | +++ | PMN and LP | + | LP and PMN | - | - | - | - | * | - |
| N4 | ++++ | PMN | +++ | PMN and LP | + | LP and PMN | + | LP | + | LP | - | - |
| C1 | +++ | PMN | ++ | PMN and LP | - | - | + | LP | - | - | * | - |
| C2 | +++ | PMN | ++ | PMN and LP | - | - | - | - | - | - | - | - |
| C3 | + | e LP | + | PMN and LP | + | LP and PMN | - | - | - | - | - | - |

| | | | | | | | | | | | | |
|---|---|---|---|---|---|---|---|---|---|---|---|---|
| Groupe: N1: PLGA:HA; PLGA:HA + 0,1% tetracycline: N3: PLA:HA; N4: PLA:HA + 0,1% tetracycline; C1: PLGA; C2: PLA ; C3: HA I.I.: inflammatory infiltrate PMN: Inflammatory infiltrate with predominance of polimorphonuclear leukocytes. LP: Inflammatory infiltrate with predominance of lymphocyte, leukocytes and plasmocytes +++ : Dense inflammatory infiltrate. ++ :Mild inflammatory infiltrate. + :Light inflammatory infiltrate - Absence of inflammatory infiltrate. * Absence of pellet at implant location. | | | | | | | | | | | | |

Depending on their mode of adherence to the matrix, the cells may have their behavior affected: strong adherence and cell propagation are associated to proliferation, while round cells (cubic or polygonal, as is the case of osteoblasts) are generally associated to specific functions (CHICUREL et al., 1998).

The pellet of the PLGA:HA + TCN 0,1% composite, removed 28 days after implantation, displayed a profile of composite degradation and the presence of rat tissue samples. The pellet of the PLA:HA + TCN 0,1% composite, also removed 28 after implantation, revealed tetracycline crystals and cell adsorption. As expected, PLGA polymer pellets revealed greater degradation due to their higher-speed degradation. Control pellets, made of PLGA polymer only, were rapidly dissolving from the 13^{th} day on, and were not identified on the 56^{th} day.

On the 56^{th} day the pellets of PLGA:HA were disintegrated and encapsulated by a conjunctive purse of conjunctive tissue, while the pellets of PLA:HA kept their rigidity, their original size and form. Control pellets, made only from the PLA polymer, had their original 7mm diameter increased, reaching an average diameter of 10mm from the 28^{th} day. Macroscopically, one PLA pellet taken from a rat on the 56^{th} day displayed two blood vessels.

In general, the microscopic findings corroborate the results of the macroscopic analysis of the studies in vivo. Therefore, we can state that all the materials tested PLGA, PLGA:HA, PLGA:HA + tcn, PLA, PLA:HA, PLA:HA + tcn - presented a good level of biocompatibility, including showing clear indications of neovascularization, as evidenced in a PLA pellet in the 56^{th} day.

***Histological evaluation of the dentine-pulp complex of rats after direct pulp capping made with composites.***

The relevance of this study is that it is focused on the development of a new biomaterial combining one bioactive bioceramics matrix with one biodegradable polymer almed at producing more effective tissue repair as compared to those achieved with other materials traditionally used in dentistry. The objective of this study was to check the inflammatory response of the pulp tissue and dentine tissue formation in rats after the use of composites made from hydroxiapatite as compared to the use of calcium hydroxiapatite (CETEA 143/04). The composites were prepared and sterilized with ethylene oxide, following the procedures described previously. 36 six-week-old rats (Rattus norvergicus) of the Wistar lineage, weighing between 250 and 300g were used in this investigation.

The animals were placed in two groups: (1) Calcium hydroxide; (2) Bioceramics HA and PLGA; (3) Negative control within four periods of time (1, 7. 15 and 30 days). The animals received an intraperitoneal anesthetic injection of 10 ml Ketamine 10% and 7.5 ml Xilazine 10% (body weight: 0.1ml/100g).

A cavity in the first upper molar oclussal face of the animals was made using a spherical drill n. 1/4 (ISO Maillefer), in total isolation, with water refrigeration and low rotation, keeping the animal in adequate equipment. No additional retention was supplied for the preparation of the cavity. Cavities and exposures were carefully cleaned with cotton balls and distilled water during four minutes. After being sacrificed, the animals underwent the histological processing of their teeth performed with the use of hematoxilin-eosin followed by a qualitative and quantitative histological analysis. The data was subjected to a ANOVA Test statistical analysis. The dentine bridge was measured with a 60-micrometer reticle.

In group (1), necrosed areas and a mild acute inflammatory process were evident after seven days. From the fourteenth day on a mild chronic infiltrate was observed, while around the thirtieth day and on that infiltrate was increasingly being reduced resulting in the formation of hard tissue. In group (2) on days 1 and 7 a mild acute infiltrate with the presence of fibrous tissue, while the formation of hard tissue started on the fourteenth day and was evident on the thirtieth day, when the obstruction of the pulp exposure accompanied with a light infiltrate was observed in 100% of the samples. In group (3) intense infiltrate is predominant, accompanied by necrosed areas resulting in the absence of mineralized tissue formation. Our conclusion is that the inflammatory Infiltrate was less intense in the composites group, allowing for dentine tissue formation and a more effective tissue repair as compared to that resulting from calcium hydroxide, and the density of the newly formed osteoid tissue closer to that of the repairing dentine.

The same technique used In the preparation and blending of calcium phosphate cements, non biodegradable polymers and carrier substances Is used for those composites in the various proportions and associations indicated for different uses, in the reconstruction and repair of the skeletal muscular system, mainly that of the bone and/or cartilage tissue.

**CLAUSES**

1. Composite preparation process made from bioceramics and biodegradable or non biodegradable polymers, cementing and/or carrier substances characterized by the inclusion of
a. Bioceramics like Ca₅(PO₄)₃OH, CaCO₃, CaSO₄, CaSO₂, Ca₃(PO₄), CaOSiO₂(NOs)₂, Ca₁₀(PO₄)6(OH)₂;
b. Biodegradable polymers like PLGA, PLA, PGA and their corresponding blends:
   - Non degradable polymers, like PMMA;
   - cementing substances and/or calcium phosphate;
   - carrier substances like sodium alginate, hydroximethyl cellulose
c. Antibiotics concentration of 0,001% to 5%, angiostensin (1-7) from 1 pmol to 50 µmol;
d. Polysaccharide encapsulated antibiotics and angiostensin (1-7);
e. Antibiotic and angiostensin (1-7) microencapsulation or nanoencapsulation in biodegradable polymers like PLGA, PLA, PGA and corresponding blends.

2. Composite preparation process made from bioceramics and biodegradable polymers and the other substances prescribed characterized by the use of bioceramics and biodegradable polymers and the other substances prescribed in the proportion 90%-10% of bioceramics and 10%-90% biodegradable polymers.

3. Composite preparation process made from bioceramics and biodegradable polymers and the other substances prescribed characterized by the use of organic solvents and water for the preparation of composites and the achievement of irregular forms of moldable composites, like stems, plates and screws.

4. Composite preparation process made from bioceramics and biodegradable polymers, and the other substances prescribed, according to claim n. 1 , characterized by the use of bioceramics like Osteosynt, calcium phosphate, hydroxiapatite and corresponding blends.

5. Composite preparation process made from bioceramics and biodegradable polymers and the other substances prescribed, according to claim n. 1, characterized by the use of antibiotics like tetracycline.

6. Composite preparation process made from bioceramics and biodegradable polymers and the other substances prescribed, according to claim n. 1, characterized by the use of polysaccharides and oligosaccharides, natural cyclodextrin like α, β, e γ, as well as the sodium alginate binding agents modified cyclodextrin like hydroxipropil-β-cyclodextrin, and corresponding blends for antibiotics and angiostensin (1-7) encapsulation.

7. Composite preparation process made from bioceramics and biodegradable or non biodegradable polymers and others, according to claim n. 1, characterized by the use of polysaccharides and oligosaccharides, natural cyclodextrin like α, β, e γ, as well as modified cyclodextrin like hydroxipropil- β-cyclodextrin, and corresponding blends for angiostensin (1-7) encapsulation.

8. Composite preparation process made from bioceramics and biodegradable or non biodegradable and others, according to claims 1, 2 and 3 characterized by the micro and nanoencapsulation of tetracycline in biodegradable or non biodegradable polymers and others, like PLGA, PLA, PGA , PMMA and calcium phosphate cements.

9. Composite preparation process made from bioceramics and biodegradable or non biodegradable polymers and others, according to claims 1 , 2 and 3 characterized by the micro e nanoencapsulation of the inclusion composites between the tetracycline and the cyclodextrin in biodegradable or non biodegradable polymers and others like PLGA, PLA and PGA, PMMA and calcium phosphate cements.

10. Composite preparation process made from bioceramics and biodegradable or non biodegradable polymers and others, according to claims 1 2 and 3 characterized by the micro and nanoencapsulation of the angiotensin (1-7) in biodegradable or non biodegradable polymers and others, like PLGA, PLA, PGA, PMMA and calcium phosphate cements.

11. Composite preparation process made from bioceramics and biodegradable or non biodegradable polymers and others, according to claims 1 , 2 and 3 characterized by the micro e nanoencapsulation of the inclusion composites of angiotensine (1-7) and the cyclodextrines in biodegradable or non biodegradable polymers and others like PLGA, PLA PGA, PMMA and calcium phosphate cements.

12. Composite preparation process made from bioceramics and biodegradable or non biodegradable polymers and others, according to claims 1, 2 and 3 characterized by its use as grafts in the recovery of bone tissue and induction of osteoid tissue.

13. Composite preparation process made from bioceramics and biodegradable polymers, according to claims 1 , 2 and 3 characterized by its use in the recovery of bone tissue and induction of osteoid tissue and support and/or repair parts of the skeletal muscular system.

14. Composite preparation process made from bioceramics and biodegradable polymers, according to claims 1 , 2 and 3 characterised by its use in the induction of compact dentine bridge and a matrix with favorable geometry for mineral deposition.

15. Product made from bioceramics and biodegradable polymers characterized by the use of:
a. Ca₅(PO₄)SOII, CaCO₃, CaSO₄, CaSO₂, Ca₃(PO₄), CaOSiO₂(NO₃)₂, Caio(PO₄)₆(OH)₂;
b. Biodegradable polymers like PLGA, PLA and PGA and corresponding blends:
   - Non degradable polymers, like PMMA;
   - cementing substances and/or calcium phosphate;
   - carrier substances like sodium alginate, hydroximethyl cellulose
c. Antibiotics concentration of 0,001% to 5%, angiostensin (1-7) from 1 pmol to 50 µmol;
d. Polysaccharide encapsulated antibiotics and angiostensin (1-7);
e. Micro and nanoencapsulation of antibiotics and angiostensin (1-7) in biodegradable polymers like the PLGA, PLA and PGA and corresponding blends.

16. Product in accordance with claim n. 15, characterized by its use in the recovery of bone tissue and induction of bone tissue.

17. Product in accordance with claim n. 15, characterized by its use in the induction of repairing dentine, formation of compact dentine bridge and a matrix with favorable geometry for mineral deposition.

## Claims

1. Composites based on bioceramics and biodegradable or non biodegradable polymers for tissue restitution comprising:
a) bioceramics selected from the group consisting of Ca₅(PO₄)₃OH, CaCO₃, CaSO₄, CaSO₂, Ca₃(PO₄), CaOSiO₂(NO₃)₂, Ca₁₀(PO₄)₆(OH)₂; and
b) biodegradable polymers selected from the group consisting of PLA, PGA, PLGA; or
c) non-biodegradable polymers, selected from the group consisting of PMMA; and
d) antibiotics, selected from the group consisting of tetracycline, dioxicycline, minocycline.

2. Composites based on bioceramics and biodegradable or non biodegradable polymers for tissue restitution, according to claim 1, wherein the concentration of the bioceramics in relation to the total weight of the composite ranges from 90% up to 10% (w/w).

3. Composites based on bioceramics and biodegradable or non biodegradable polymers for tissue restitution, according to claims 1 and 2, wherein the concentration of the polymers in relation to the total weight of the composite ranges from 10% up to 90% (w/w).

4. Composites based on bioceramics and biodegradable or non biodegradable polymers for tissue restitution, according to claims 1 to 3, wherein the concentration of the antibiotics in relation to the total weight of the composite ranges from 0.001% to 5% (w/w).

5. Composites based on bioceramics and biodegradable or non biodegradable polymers for tissue restitution, according to claims 1 to 4, wherein the composites are moldable

6. Composites based on bioceramics and biodegradable or non biodegradable polymers for tissue restitution, according to claim 5,
presenting irregular shapes, selected from the group comprising stems, plates and screws.

7. Composites based on bioceramics and biodegradable or non biodegradable polymers for tissue restitution, according to claims 1 to 6, comprising optionally:
a) Cementing substances selected from the group consisting of calcium phosphate; and/or
b) Carrier substances selected from the group consisting of sodium alginate, cyclodextrins and hydroxymethyl cellulose.

8. Use of bioceramics and biodegradable or non biodegradable polymers in the manufacture of composites, as defined in claims 1 to 7, for the recovery of bone tissue, induction of osteoid tissue neoformation and support and/or repair of parts of the skeletal muscular system.

9. Use of bioceramics and biodegradable or non biodegradable polymers in the manufacture of composites, as defined in claims 1 to 8, for the induction of compact dentine bridge and a matrix with favorable geometry for mineral deposition.

10. Use of bioceramics and biodegradable or non biodegradable polymers in the manufacture of composites, as defined in claims 1 to 9, for use as grafts, for the recovery of bone tissue and induction of osteoid tissue formation and/or repair of parts of the skeletal muscular system.
